# EUROPÄISCHE PATENTANMELDUNG

(11) **EP 2 988 126 A1**
(43) Veröffentlichungstag der Anmeldung: **24.02.2016**
(21) Anmeldenummer: 15181198.1
(22) Anmeldetag: 17.08.2015
(51) Int. Cl.: G01N 33/487, A61B 5/157

(54) **EINRICHTUNG ZUR AUFBEWAHRUNG VON UTENSILIEN ZUR BLUTZUCKERMESSUNG FÜR DIABETIKER**

(30) Priorität: 19.08.2014 DE 102014111791; 19.08.2014 DE 202014103830 U
(71) Anmelder: IME-DC GmbH International Medical Equipment - Diabetes Care, 95030 Hof (DE)
(72) Erfinder: Kamalak, Serif, 95032 Hof (DE)
(74) Vertreter: Sperschneider, Alexandra

(57) **Zusammenfassung**

Die vorliegende Erfindung betrifft eine Einrichtung (10) zur Aufbewahrung von Utensilien zur Blutzuckermessung für Diabetiker, wobei die Utensilien ein Blutzuckermessgerät (12) mit einer Aufnahme für Teststreifen, eine Stechhilfe (14) mit auswechselbaren Lanzetten und ein Behältnis (16) für Teststreifen umfassen. Die Einrichtung (10) weist Bereiche zur Aufnahme der Utensilien auf, wobei die Utensilien in den Bereichen mit der Einrichtung (10) lösbar befestigt und die Utensilien über mindestens eine Seite der Einrichtung (10) funktional zugänglich sind. Die Einrichtung (10) weist an der mindestens einen Seite Zugangsbereiche auf, die im Wesentlichen kongruent zu Zuführ- und Entnahmebereichen der Utensilien sind und eine Bedienung der Utensilien ohne eine Entnahme der Utensilien ermöglichen.

## Beschreibung

Einrichtung zur Aufbewahrung von Utensilien zur Blutzuckermessung für Diabetiker.

Die vorliegende Erfindung betrifft eine Einrichtung zur Aufbewahrung von Utensilien zur Blutzuckermessung für Diabetiker. Solche Utensilien umfassen ein Blutzuckermessgerät, das eine Aufnahme für Teststreifen aufweist, eine Stechhilfe mit auswechselbaren Lanzetten und ein Behältnis für Teststreifen. Die Einrichtungen zur Aufbewahrung der Utensilien ermöglichen den Transport dieser, wobei die Utensilien für einen Diabetiker schnell greifbar und leicht bedienbar sein sollen. Zudem sollen durch solche Einrichtungen die Utensilien beim Transport geschützt werden. Die Einrichtungen können hierzu beispielsweise vor Stößen, hohen oder niedrigen Temperaturen und Feuchtigkeit schützen.

Aus dem Stand der Technik ist eine Vielzahl von Einrichtungen zur Aufbewahrung von Utensilien zur Blutzuckermessung bekannt. Die Einrichtungen bestehen in der Regel aus einem flexiblen Material, z. B. einem Textil oder einem flexiblen Kunststoff, und weisen Aufnahmen für die Utensilien auf. Insbesondere sind die Einrichtungen so ausgebildet, dass sie beispielsweise über einen Reißverschluss geöffnet und auseinandergeklappt werden können, so dass die Utensilien für einen Benutzer zugänglich sind. Hierbei ist es erforderlich, dass die Einrichtung auf einer Fläche abgelegt wird, damit der Diabetiker mit beiden Händen eine Blutzuckermessung durchführen kann. Wird die Einrichtung auf einem unebenen Untergrund angeordnet, verrutschen die Einrichtungen, was zu einer erschwerten Bedienung der Utensilien führt.

Aus AT 004 359 U1 ist ein raumoptimiertes Utensilienetui für Diabetiker bekannt. Die darin beschriebene Einrichtung zur Aufbewahrung von Utensilien zur Blutzuckermessung, -behandlung und -dokumentation für Diabetiker umfasst mehrere klappbare Segmente mit Befestigungselementen für die Utensilien. Die Segmente sind in einer allgemeinen ebenen Offenlage ausklappbar und im zusammengeklappten Zustand mittels einer Verschließeinrichtung zu einem Etui verschließbar. In der Einrichtung ist sämtlichen Utensilien ein Platz an den Segmenten zugeordnet. Auch bei der in AT 004 359 U1 beschriebenen Einrichtung ist es erforderlich, dass das Etui geöffnet wird bevor dann ein Diabetiker die Blutzuckermessung durchführen kann, wobei es nicht möglich ist, bspw. im Stehen eine Blutzuckermessung durchzuführen, ohne die Einrichtung auf einen Untergrund abzulegen.

Nachteilig bei den Einrichtungen aus dem Stand der Technik ist, dass bei allen bekannten Ausführungen die Einrichtungen oder das Etui auf einer ebenen Fläche abgelegt werden müssen, die Einrichtung oder das Etui geöffnet werden muss und anschließend erst die Blutzuckermessung durchgeführt werden kann. Ein einfaches, sicheres und schnelles Blutzuckermessen, beispielsweise im Stehen ohne die Einrichtung abzulegen, ist bei den Einrichtungen aus dem Stand der Technik nicht möglich.

Aufgabe der vorliegenden Erfindung ist es daher, eine Einrichtung zur Aufbewahrung von Utensilien zur Blutzuckermessung für Diabetiker anzugeben, wobei ein einfaches und sicheres Messen ermöglicht wird, wobei die erforderlichen Bedienschritte für eine Blutzuckermessung gegenüber aus dem Stand der Technik bekannten Einrichtungen erheblich reduziert sind und der Benutzer auch im Stehen eine Blutzuckermessung durchzuführen, ohne die Einrichtung anlegen zu müssen.

Die Aufgabe wird durch eine Einrichtung mit den in Anspruch 1 angegebenen technischen Merkmalen gelöst.

Vorteilhafte Weiterbildungen der Erfindung sind in den Unteransprüchen im Detail angegeben.

Bei einer erfindungsgemäßen Einrichtung zur Aufbewahrung von Utensilien zur Blutzuckermessung für Diabetiker, wobei die Utensilien mindestens ein Blutzuckermessgerät mit einer Aufnahme für Teststreifen, eine Stechhilfe mit auswechselbaren Lanzetten und ein Behältnis für Teststreifen umfassen, weist die Einrichtung Bereiche zur Aufnahme mindestens der vorgenannten Utensilien auf, wobei die Utensilien in den Bereichen mit der Einrichtung lösbar befestigt und die Utensilien über mindestens eine Seite der Einrichtung funktional zugänglich sind, wobei die Einrichtung an der mindestens einen Seite Zugangsbereiche aufweist, die im Wesentlichen kongruent zu Zuführ- und Entnahmebereichen der Utensilien sind, und eine Bedienung der Utensilien ohne eine Entnahme der Utensilien ermöglichen.

Die Einrichtung ermöglicht eine Blutzuckermessung ohne dass die Utensilien aus der Einrichtung entnommen werden müssen oder dass die Einrichtung geöffnet werden muss. Die Einrichtung ermöglicht daher eine Blutzuckermessung auf einfache und sichere Art und Weise, wobei über die Zugangsbereiche beispielsweise Teststreifen aus einem Behältnis entnommen werden können, ohne die Einrichtung zu öffnen oder das Behältnis der Einrichtung zu entnehmen, und die Teststreifen in die Aufnahme des Blutzuckermessgerätes einzusetzen ohne das Blutzuckermessgerät aus der Einrichtung entnehmen zu müssen oder die Einrichtung zu öffnen. Ebenso kann die Stechhilfe zur Blutgewinnung bedient werden, ohne die Stechhilfe der Einrichtung entnehmen zu müssen oder die Einrichtung zu öffnen.

Die Einrichtung ermöglicht daher auch ein Blutzuckermessen im Stehen, wobei ein Diabetiker mit der einen Hand die Einrichtung hält und mit der anderen die Utensilien verwenden bzw. bedienen kann. Es ist hierbei nicht wie im Stand der Technik notwendig, die Einrichtung auf einer ebenen Fläche abzulegen und die Einrichtung zu öffnen, um ein Messen durchzuführen.

Die Zugangsbereiche ermöglichen neben einem Entnehmen von Teststreifen und dem Einsetzen der Teststreifen in das Blutzuckermessgerät, ein Auswechseln der Lanzetten für die Stechhilfe sowie ein Spannen und Auslösen für die Stechhilfe. Hierzu umfasst die Einrichtung die Zugangsbereiche. Die Utensilien sind über die Bereiche fest in Position zu der Einrichtung angeordnet. Dazu sind die Bereiche entsprechend ausgebildet und verhindern, dass die Utensilien verrutschen können.

Die Einrichtung kann ein Aufnahmeteil und eine Abdeckung aufweisen und die Bereiche zur Aufnahme der Utensilien können in dem Aufnahmeteil angeordnet sein, wobei das Aufnahmeteil und die Abdeckung mindestens abschnittsweise lösbar miteinander verbunden und die Utensilien über Aufnahmen in dem Aufnahmeteil befestigt sind, wobei die Zugangsbereiche in Wänden der Aufnahmen des Aufnahmeteils und/oder der Abdeckung angeordnet sind. Die Abdeckung kann von dem Aufnahmeteil in diesen Ausführungen entnommen werden, um beispielsweise beim erstmaligen Benutzen der Einrichtung die Utensilien einzusetzen oder die Utensilien auszutauschen. Das Blutzuckermessgerät muss auch in regelmäßigen Abständen entnommen werden, um die Batterien auszutauschen. Verfügt das Blutzuckermessgerät über einen wieder aufladbaren Akkumulator, so kann die Einrichtung einen Zugangsbereich für ein sogenanntes Ladekabel bzw. für einen Adapter des Ladekabels aufweisen, so dass ohne die Einrichtung zu öffnen oder ohne eine Entnahme des Blutzuckermessgerätes der Akkumulator des Blutzuckermessgerätes aufgeladen werden kann. Das Aufnahmeteil und die Abdeckung können hierbei mindestens abschnittsweise lösbar miteinander verbunden sein, wobei die Abdeckung vollständig von dem Aufnahmeteil entnommen werden kann oder in weiteren Ausführungen fest mit dem Aufnahmeteil verbunden ist und sich nur bereichsweise von dem Aufnahmeteil entfernen lässt. Die Bereiche zur Aufnahme der Utensilien sind in dem Aufnahmeteil angeordnet, wobei das Aufnahmeteil entsprechende Aufnahmen hierfür aufweist. Die Aufnahmen sind so ausgebildet, dass die Utensilien in den Aufnahmen ihre Position zu der Einrichtung und zu den Zugangsbereichen beibehalten.

Das Aufnahmeteil kann aus Kunststoff bestehen oder als Kunststoffvoll- oder -hohlkörper ausgebildet sein und schalenförmige Aufnahmen für die Utensilien aufweisen, die darin klemmend haltbar sind. Derartig ausgebildete Aufnahmeteile sind widerstandsfähig gegenüber mechanischen Einwirkungen (Stöße, Druck). In die Kunststoffschale oder in den Kunststoffvoll- oder -hohlkörper können die Aufnahmen für die Utensilien eingebracht sein.

Das Aufnahmeteil kann eine ebene Grundfläche und eine der höchsten Utensilie entsprechende Höhe oder eine inhomogene Oberfläche mit einer den Höhen der Utensilien angepassten Abstufung aufweisen. Das Aufnahmeteil ist dementsprechend so hoch bzw. weist eine entsprechende Dicke auf, dass sämtliche Utensilien vollständig von dem Aufnahmeteil umgeben oder darin aufgenommen sind. Die weiteren Abmaße des Aufnahmeteils sind entsprechend den Utensilien ausgelegt, so dass im Wesentlichen alle Utensilien in einer Kunststoffschale oder einem Kunststoffvoll- bzw. -hohlkörper aufgenommen bzw. angeordnet sein können. Jedoch können in weiteren Ausführungen zumindest Abschnitte der Utensilien aus der Kunststoffschale oder dem Kunststoffvoll- bzw. -hohlkörper oder dem Aufnahmeteil herausstehen.

Das Aufnahmeteil und/oder die Abdeckung können auch aus einem flexiblen Material bestehen oder die Wände der schalenförmigen Aufnahmen können federelastisch ausgeführt sein. Flexible Materialien sind beispielsweise Textilien aus Kunst- oder Naturfasern. Als Material für ein Aufnahmeteil mit federelastischen Aufnahmen können zum Beispiel Schaumstoffe verwendet werden. Schaumstoffe können die Utensilien sicher in den Aufnahmen halten und verhindern ein Herausbewegen der Utensilien. Hierbei können die Aufnahmen auch geringfügig kleiner ausgebildet sein als die Utensilien selbst. In weiteren Ausführungen können Aufnahmeteile aus einer Kunststoffschale oder als Kunststoffvoll- bzw. -hohlkörper ausgebildete Aufnahmeteile flexible Materialien aufweisen, die die Aufnahmeteile umgeben und aus dekorativen Zwecken verwendet werden. Gleiches gilt für die Abdeckung. Ein Material für eine dekorative Umhüllung ist beispielsweise Leder.

Bestehen das Aufnahmeteil und/oder die Abdeckung aus einem flexiblen Material, so kann das Aufnahmeteil mindestens eine Verstärkung aufweisen, so dass die Utensilien in den Bereichen ihre Position zu den Zugangsbereichen der Einrichtung beibehalten. Durch eine Verstärkung lässt sich eine Einrichtung aus flexiblem Material auch leichter zur Blutzuckermessung verwenden.

Das Aufnahmeteil und/oder die Abdeckung können Befestigungsmittel und/oder Aufnahmevertiefungen für die Utensilien aufweisen. Die Befestigungsmittel und/oder die Aufnahmevertiefungen dienen hierbei als Aufnahmen für Utensilien. Befestigungsmittel können beispielsweise auch Gummibänder, Rastelemente oder Klemmteile sein, die die Utensilien zumindest abstandsweise umgreifen. Im eingesetzten Zustand der Utensilien halten dann die Befestigungsmittel die Utensilien sicher in Position. Aufnahmevertiefungen können beispielsweise in einem Kunststoffvoll- oder -hohlkörper vorgesehen sein. Die Aufnahmevertiefungen entsprechen dann den äußeren Maßen der Utensilien so dass die Utensilien vollständig oder auch nur abschnittsweise in den Aufnahmevertiefungen aufgenommen werden können. Hierzu weisen die Aufnahmevertiefungen Zugänge oder freie Bereiche auf, die zu den Zugangsbereichen der Einrichtung führen und den Zugang zu den Utensilien hierüber freigeben.

Das Aufnahmeteil und/oder die Abdeckung können eine Öffnung aufweisen, über welche im geschlossenen Zustand der Einrichtung mindestens eine Anzeige des Blutzuckermessgerätes sichtbar ist. Beispielsweise weist die Abdeckung eine Öffnung für ein Display des Blutzuckermessgerätes auf. Ferner kann das Aufnahmeteil an seiner unteren, der Abdeckung gegenüberliegenden Seite ein Sichtfenster aufweisen, über welches Anzeigen, Hinweise oder sonstige Informationen des Blutzuckermessgerätes, der Stechhilfe oder des Behältnisses für Teststreifen sichtbar sind. Die Öffnungen können zudem eine transparente Folie aufweisen, die das Eindringen von Staub und anderen Verunreinigungen in die Einrichtung verhindert, aber dennoch den Blick auf die Utensilien freigibt.

Die Einrichtung kann Befestigungsmittel aufweisen, über welche die Einrichtung an Textilien oder Gegenständen lösbar befestigbar ist. Über die Befestigungsmittel kann beispielsweise die Einrichtung an einem Gürtel befestigt werden. Für eine solche Befestigung eignet sich beispielsweise ein als Klipp bezeichnetes Befestigungsmittel.

Die Größe und der Umfang der Aufnahmevertiefungen können jeweils an das Maß und den Umfang einer der Utensilien angepasst sein. In einer ersten Gruppe von Ausführungen der Einrichtung sind die Aufnahmevertiefungen so ausgebildet, dass die Utensilien teilweise aus den Aufnahmevertiefungen herausragen. In einer zweiten Gruppe von Ausführungen der Einrichtung sind die Aufnahmevertiefungen so ausgebildet, dass die Utensilien vollständig in den Aufnahmevertiefungen aufgenommen sind. In einer weiteren Gruppe von Ausführungen der Einrichtung bestehen das Aufnahmeteil und die Abdeckung jeweils aus einer Kunststoffschale, einem Kunststoffvollkörper oder Kunststoffhohlkörper mit Aufnahmevertiefungen, wobei das Aufnahmeteil und die Abdeckung jeweils einen Teil der Utensilien in ihren Aufnahmevertiefungen aufnehmen. Das Aufnahmeteil und die Abdeckung sind in diesen Ausführungen ähnlich aufgebaut.

Das Aufnahmeteil und die Abdeckung können über einen Magnetverschluss, eine Rastverbindung, einen Klettverschluss oder einen Reißverschluss mindestens abschnittsweise miteinander verbindbar sein.

Hierbei können das Aufnahmeteil und die Abdeckung verschwenkbar miteinander verbunden sein, vorzugsweise sind das Aufnahmeteil und die Abdeckung an einer Seite bzw. einer das Aufnahmeteil und die Abdeckung verbindenden Kante verschwenkbar (z. B. über ein Filmscharnier). Die übrigen Abschnitte des Aufnahmeteils und der Abdeckung sind dann über beispielsweise einen Reißverschluss miteinander verbindbar. Jedoch kann auch an der Abdeckung und auch an dem Aufnahmeteil eine Einrichtung vorgesehen sein, die einen Magnetverschluss umfasst. Alternativ kann auch abschnittsweise ein Klettverschluss sich über einen bestimmten Abschnitt erstrecken. Auch Rastverbindungen können hierbei verwendet werden. Rastverbindungen eignen sich auch insbesondere bei Ausführungen, wobei die Abdeckung vollständig von dem Aufnahmeteil entfernt werden kann.

Die Zugangsbereiche können Öffnungen, Ausnehmungen oder Durchbrüche sein. Öffnungen können beispielsweise in dem Aufnahmeteil vorgesehen sein. Ausnehmungen können beispielsweise an den Kanten des Aufnahmeteils und der Abdeckung vorgesehen sein, so dass durch gegenüberliegende Ausnehmungen des Aufnahmeteils und der Abdeckung Öffnungen als Zugangsbereiche für die Einrichtung bereitgestellt werden.

Die Einrichtung kann zusätzliche Bereiche zur Aufnahme weiterer Utensilien aufweisen. Beispielsweise können hierüber Lanzetten, ein Insulin-Pen oder auch Pen-Nadeln sowie Patronen für Pens vorgesehen und über Zugangsbereiche zugänglich sein.

Die weitere Utensilie kann, wie zuvor schon angesprochen, auch ein Insulinspritzgerät (Pen) sein, das ohne eine Schutzkappe in eine röhrenförmige Aufnahme der Einrichtung einsteckbar und aus dieser wahlweise wieder herausziehbar ist, und das mit einer Schutzkappe mindestens mit dieser in einer Aufnahme einklemmbar eingelegt ist und aus der Schutzkappe wahlweise herausziehbar ist. In der Regel weisen stabförmige Insulinspritzgeräte eine Schutzkappe auf, die über den Gehäusebereich schützend schiebbar ist, in welchem Bereich sich die Insulinpatrone befindet, an der auch eine Subkutaninjektionsnadel befestigt sein kann. In der einen Ausführung wird also mindestens die Schutzkappe durch Klemmung gesichert gehalten, so dass das Insulinspritzgerät über eine Stirnseite der Einrichtung vorsteht oder mit dieser endet, zumindest aber hierüber herausgezogen werden kann. Wenn eine röhrenförmige Aufnahme vorgesehen ist, können die Insulinspritzgeräte auch direkt eingesteckt werden. In jedem Fall ist die Haftung innerhalb der Aufnahme oder innerhalb der Schutzkappe so bemessen, dass das Insulinspritzgerät herausziehbar ist, wenn es für die Verabreichung einer Insulinspritze benötigt wird.

An der Außenseite der Einrichtung können auch Fächer zur Aufnahme von Unterlagen vorgesehen sein.

Weitere Vorteile, Merkmale sowie Ausgestaltungsmöglichkeiten ergeben sich aus der nachfolgenden Figurenbeschreibung von nicht einschränkend zu verstehenden Ausführungsbeispielen.

In den Zeichnungen zeigen:
- Fig. 1: eine schematische Draufsicht auf ein Aufnahmeteil einer Einrichtung;
- Fig. 2: eine schematische Draufsicht auf eine Abdeckung der Einrichtung von Fig. 1;
- Fig. 3: eine schematische Draufsicht auf eine Aufnahmeschale;
- Fig. 4: eine schematische Draufsicht auf eine weitere Aufnahmeschale;
- Fig. 5: eine schematische Darstellung einer Einrichtung im geöffneten Zustand in perspektivischer Ansicht;
- Fig. 6: eine schematische Darstellung der Einrichtung von Fig. 5 im geschlossenen Zustand in perspektivischer Ansicht;
- Fig. 7: eine schematische Darstellung einer Aufnahmeschale gemäß einer weiteren Ausführung in perspektivischer Ansicht;
- Fig. 8: eine schematische Darstellung einer weiteren Einrichtung im geöffneten Zustand in perspektivischer Ansicht;
- Fig. 9: eine schematische Darstellung einer noch weiteren Einrichtung im geöffneten Zustand in perspektivischer Ansicht;
- Fig. 10: eine schematische Darstellung einer Ausführungsvariante einer Einrichtung im geöffneten Zustand der Ausführung von Fig. 9 in perspektivischer Ansicht; und
- Fig. 11: eine schematische Darstellung einer weiteren Ausführungsform einer Einrichtung im geöffneten Zustand in perspektivischer Ansicht.

In den Figuren mit gleichen Bezugszeichen versehene Teile und Komponenten entsprechen im Wesentlichen einander, solange nichts anderes angegeben ist.

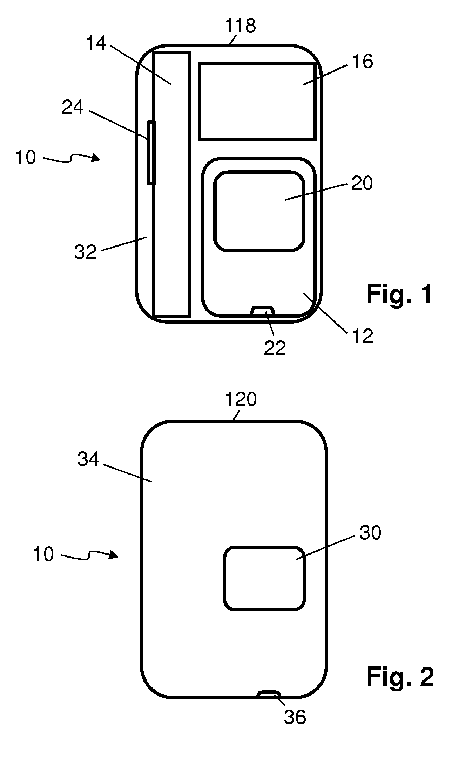

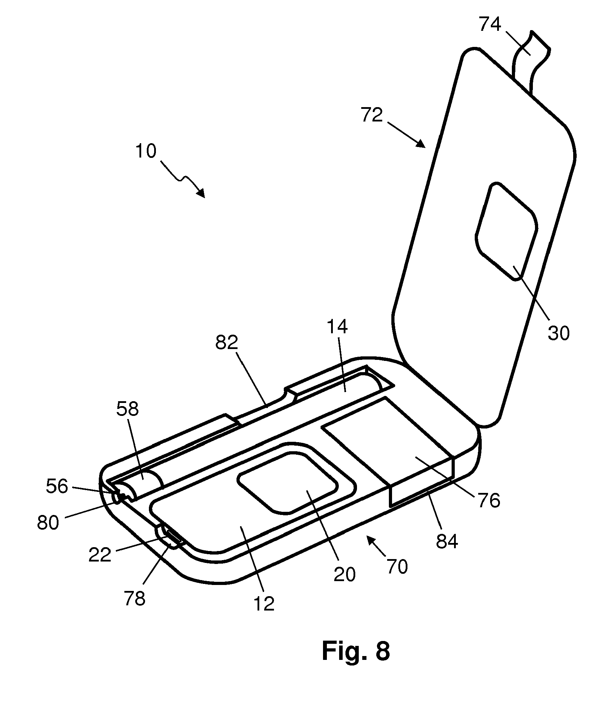

Fig. 1 zeigt eine schematische Draufsicht auf ein Aufnahmeteil einer Einrichtung 10. Die mit dem Aufnahmeteil verbundene Abdeckung 34 der Einrichtung 10 ist in Fig. 2 dargestellt. Das Aufnahmeteil und die Abdeckung 34 sind über ein Filmscharnier an den Kanten 118 und 120 miteinander verschwenkbar verbunden. Im geschlossenen Zustand der Einrichtung 10 liegt die Abdeckung 34 auf dem Aufnahmeteil auf. Die Abdeckung 34 und das Aufnahmeteil können beispielsweise über ein Verschlusselement 74 (siehe Fig. 8, 9 und 10) miteinander verbunden sein. Das Verschlusselement 74 weist einen Magneten auf, der mit einem Metallteil oder einem korrespondierenden Magneten an dem Aufnahmeteil zusammenwirkt, wobei das Verschlusselement 74 an der Abdeckung 34 angeordnet ist. Über ein Verschlusselement 74 kann die Einrichtung 10 verschlossen werden.

Das Aufnahmeteil von Fig. 1 besteht aus einem Kunststoffschalenkörper. In dem Kunststoffschalenkörper sind ein Blutzuckermessgerät 12, eine Stechhilfe 14 und ein Behältnis 16 für Teststreifen aufgenommen. Das Blutzuckermessgerät 12 weist ein Display 20 und eine Zuführöffnung 22 auf. Über die Zuführöffnung 22 können Teststreifen aus dem Behältnis 16 eingesetzt werden. Das Blutzuckermessgerät 12 kann zudem Tasten zur Bedienung des Blutzuckermessgerätes 12 aufweisen. Die Stechhilfe 14 weist ein Bedienteil 24 auf, über welches die Stechhilfe 14 gespannt und ausgelöst werden kann. Hierzu kann in die Stechhilfe 14 eine Lanzette eingesetzt werden, wobei über das Bedienteil 24 ein Auslösen der Stechhilfe 14 erfolgt. Über die Stechhilfe 14 kann Blut, beispielsweise von dem Finger eines Diabetikers entnommen werden, indem über die Lanzette eine kleine Wunde zugefügt wird. Das aus der Wunde austretende Blut wird auf einen Abschnitt des Teststreifens gebracht. Der Teststreifen ist über ein erstes Ende in der Zuführöffnung 22 des Blutzuckermessgerätes 12 aufgenommen und an dem zweiten Ende befindet sich der Abschnitt zur Blutaufnahme. Das Blutzuckermessgerät 12 führt dann eine Messung des Blutzuckerwertes durch und zeigt den Wert über das Display 20 an.

Damit im geschlossenen Zustand der Einrichtung 10 das Messergebnis über das Display 20 des Blutzuckermessgerätes 12 sichtbar ist, weist die Abdeckung 34 ein Fenster 30 auf. Das Fenster 30 gibt im geschlossenen Zustand der Einrichtung 10 den Blick auf das Display 20 frei. Zumindest ist das Fenster 30 so ausgebildet, dass der Wert der Blutzuckermessung ablesbar ist. Um zu verhindern, dass Verunreinigungen über das Fenster 30 in das Innere der Einrichtung 10 gelangen, kann eine transparente Folie im Fenster 30 oder am Fenster 30 angebracht sein.

Bei Blutzuckermessgeräten 12, die einen sogenannten Touchscreen aufweisen, kann die Folie so ausgebildet sein, dass über die Folie eine wirksame Steuerung und Bedienung des Blutzuckermessgerätes 12 erfolgen kann.

Die zur Blutzuckermessung erforderlichen Utensilien (Blutzuckermessgerät 12, Stechhilfe 14 und Behältnis 16) sind fest mit den Aufnahmeteilen in Bezug auf ihre Position angeordnet. "Fest" bedeutet, dass diese Utensilien jedoch auch wieder manuell entnommen werden können, wenn die Einrichtung 10 geöffnet wird.

Die Abdeckung 34 weist mehrere Zugangsbereiche an dem Aufnahmeteil und der Abdeckung 34 auf, die so angeordnet sind, dass im geschlossenen Zustand der Einrichtung 10 ein Benutzer Testreifen aus dem Behältnis 16 entnehmen kann, den Teststreifen in die Zuführöffnung 22 des Blutzuckermessgerätes 12 einsetzen und eine Lanzette in die Stechhilfe 14 einsetzen und die Stechhilfe 14 betätigen kann. Darüber hinaus kann die Abdeckung 34 Zugangsbereiche für Tasten eines Blutzuckermessgerätes 12 aufweisen.

In Fig. 2 ist schematisch ein Zugangsbereich für die Teststreifen zu der Zuführöffnung 22 dargestellt. Hierzu dient eine Zugangsöffnung 36 zur Zuführung der Teststreifen zu der Zuführöffnung 22 des Blutzuckermessgerätes 12. Anders als in den Fig. 1 und 2 dargestellt, weist die Einrichtung 10 auch Zugangsöffnungen für das Bedienteil 24 der Stechhilfe 14 und den vorderen Abschnitt der Stechhilfe 14 zum Einsetzen von Lanzetten in die Stechhilfe 14 und eine Zugangsöffnung zur Entnahme von Teststreifen aus dem Behältnis 16 auf.

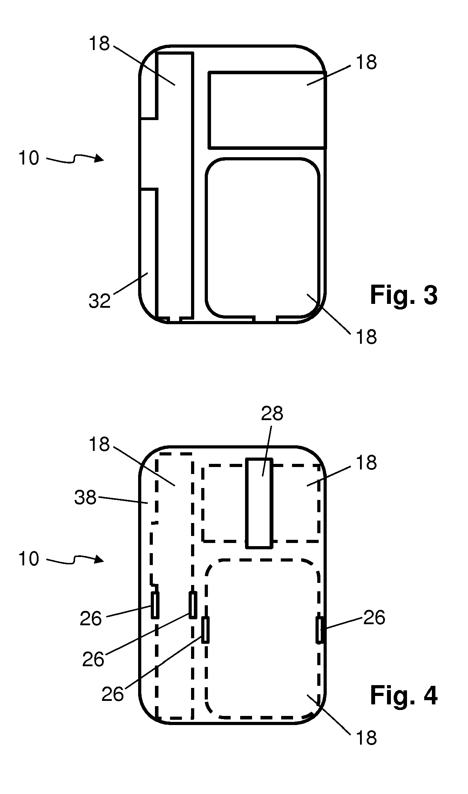

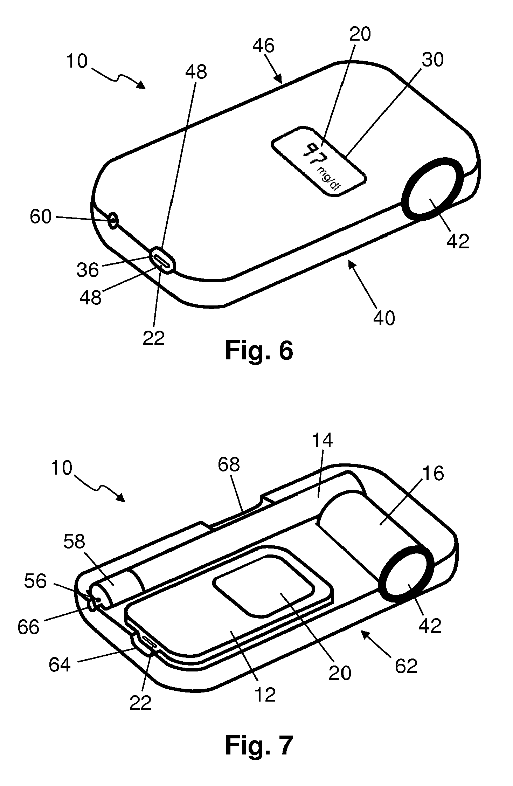

Fig. 3 zeigt eine schematische Draufsicht auf eine Aufnahmeschale 32 einer Einrichtung 10. Die Aufnahmeschale 32 ist als Vollkunststoffschale ausgebildet. Alternativ hierzu könnte die Kunststoffschale innen hohl ausgeführt sein oder Versteifungselemente aufweisen. Die Aufnahmeschale 32 weist Bereiche 18 auf, die als Aufnahmen für die Utensilien (Blutzuckermessgerät 12, Stechhilfe 14 und Behältnis 16) ausgebildet sind. Die Aufnahmen in den Bereichen 18 entsprechen daher im Wesentlichen der Form des Blutzuckermessgerätes, der Stechhilfe 14 und Behältnisses 16. Ferner sind die Aufnahmen so ausgebildet, dass die Utensilien vollständig darin aufgenommen werden können. "Vollständig aufgenommen" bedeutet in diesem Zusammenhang, dass die Utensilien nicht eine obere Fläche der Aufnahmeschale 32 überstehen. In alternativen Ausführungsformen einer Einrichtung 10 sind die Utensilien jedoch nur teilweise in den Aufnahmen aufgenommen (siehe bspw. Fig. 7). Hierbei könnte beispielsweise eine Aufnahmeabdeckung entsprechend einer Aufnahmeschale ausgebildet sein, so dass in geschlossenem Zustand der Einrichtung 10 die Utensilien in den Aufnahmen des Aufnahmeteils und den Aufnahmen der Abdeckung vollständig aufgenommen sind, wobei Zugangsbereiche frei liegen, so dass im geschlossenen Zustand der Einrichtung 10 die Utensilien funktional zugänglich sind.

Die Aufnahmen 18 der Einrichtung 10 von Fig. 3 erstrecken sich abschnittsweise bis zum Rand der Aufnahmeschale 32. Die Aufnahme 18 für das Blutzuckermessgerät 12 erstreckt sich im Bereich der Zuführöffnung 22 bis zum Rand der Aufnahmeschale 32 und die Aufnahme 18 für die Stechhilfe 14 erstreckt sich im Bereich des Bedienteils 24 und der Lanzettenöffnung ebenfalls bis zum Rand der Aufnahmeschale 32. Über die so gebildeten Zuführbereiche können das Blutzuckermessgerät 12 und die Stechhilfe 14 auch im geschlossenen Zustand der Einrichtung 10 bedient werden. Die Aufnahme 18 für das Behältnis 16 erstreckt sich auf der rechten Seite bis zum Rand der Aufnahmeschale 32.

Fig. 4 zeigt eine schematische Draufsicht auf eine weitere Aufnahmeschale, wobei in den Bereichen 18 zur Aufnahme der Utensilien (Blutzuckermessgerät 12, Stechhilfe 14 und Behältnis 16) Halteteile 26 und ein Gummiband 28 vorgesehen sind. Die Anordnung und die Anzahl der Halteelemente 26 sowie des Gummibandes 28 sind rein illustrativ und können von der in Fig. 4 gezeigten Ausführung abweichen. Bei der in Fig. 4 gezeigten Aufnahmeschale sind die Halteteile 26 und das Gummiband 28 an einem Aufnahmeboden 38 angeordnet.

Bei den in Fig. 3 und 4 gezeigten schematischen Ansichten von Aufnahmeschalen weisen diese Zugangsbereiche auf, die einen Zugang zu den Utensilien und eine Bedienung dieser für eine Blutzuckermessung ermöglichen, wenn die Einrichtung 10 geschlossen ist. Die in den Fig. 1 bis 4 gezeigten Ansichten sind rein illustrativ, so dass die Abstände der Utensilien zueinander und zu den Seitenwänden der Abdeckungen bzw. Aufnahmeteile und Aufnahmeschalen variieren können. Auch die Zugangsbereiche (z. B. Zugangsöffnungen 36) sind bei allen gezeigten Ausführungen vorgesehen.

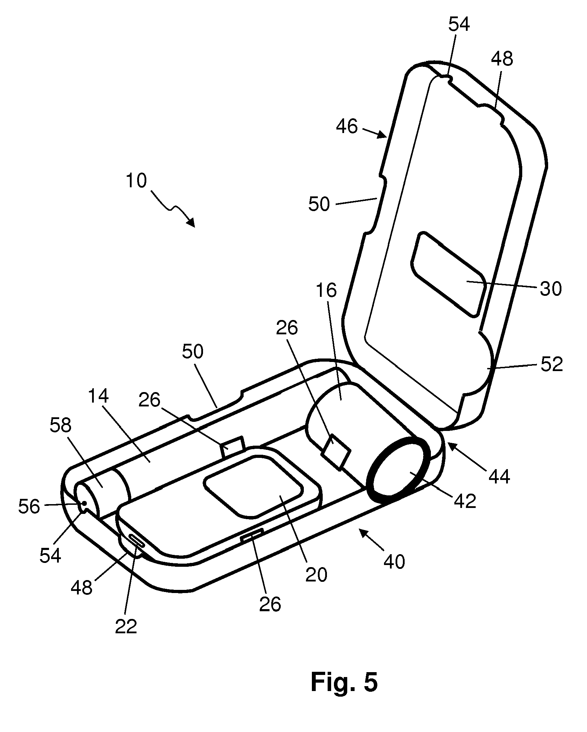

Fig. 5 zeigt eine schematische Darstellung einer Einrichtung 10 im geöffneten Zustand in perspektivischer Ansicht. Die Einrichtung 10 besteht aus einer Aufnahmeschale 40 und einer Abdeckung 46, die über ein Filmscharnier 44 miteinander verschwenkbar verbunden sind. Im geschlossenen Zustand der Einrichtung 10 (siehe Fig. 6) liegt die Abdeckung 46 auf der Aufnahmeschale 40 auf und die Aufnahmeschale 40 und die Abdeckung 46 werden beispielsweise über ein Verschlusselement 74 (in Fig. 5 und 6 nicht dargestellt) verschlossen.

Die Abdeckung 46 weist ein Fenster 30 auf, welches im geschlossenen Zustand der Einrichtung 10 den Blick auf das Display 20 des Blutzuckermessgerätes 12 freigibt. Das Blutzuckermessgerät 12, die Stechhilfe 14 und das Behältnis 16 sind über Halteteile 26 fest und in Position mit der Aufnahmeschale 40 verbunden. Ein Deckel 42 des Behältnisses 16 steht aus einem Einschnitt (in Fig. 5 nicht bezeichnet) hervor und kann selbst im geschlossenen Zustand der Einrichtung 10 geöffnet werden, um aus dem Behältnis 16 Teststreifen zu entnehmen. Um die Teststreifen in die Zuführöffnung 22 des Blutzuckermessgerätes 12 im geschlossenen Zustand der Einrichtung 10 einzusetzen, weisen die Aufnahmeschale 40 und die Abdeckung 46 korrespondierende Einschnitte 48 auf. Die korrespondierenden Einschnitte 48 bilden im geschlossenen Zustand der Einrichtung 10 eine Zugangsöffnung 36 (siehe Fig. 6). Ebenso bilden der in Fig. 5 nicht bezeichnete Einschnitt der Aufnahmeschale 40 für das Behältnis 16 und der Einschnitt 52 der Abdeckung 46 eine Zugangsöffnung, über die im geschlossenen Zustand der Einrichtung 10 Teststreifen aus dem Behältnis 16 entnommen werden können. Der Einschnitt 50 der Aufnahmeschale 40 und der Einschnitt 50 der Abdeckung 46 bilden eine weitere Zugangsöffnung für das Bedienteil 24 (in Fig. 5 nicht dargestellt) der Stechhilfe 14. Ferner weisen die Aufnahmeschale 40 einen Einschnitt 54 und die Abdeckung 46 einen Einschnitt 54 auf, die ebenfalls wieder eine Zugangsöffnung bilden, wobei über diese Zugangsöffnung eine Lanzette zur Gewinnung von Bluttropfen aus der so gebildeten Zugangsöffnung hervortritt. Die Stechhilfe 14 weist eine vordere Aufnahme 58 mit einer Öffnung 56 auf, wobei die Aufnahme 58 zum Einsetzen von Lanzetten abgenommen werden kann. Über die Öffnung 56 tritt die Spitze der Lanzette bei einer Betätigung über das Bedienteil 24 hervor.

Fig. 6 zeigt eine schematische Darstellung der Einrichtung 10 von Fig. 5 im geschlossenen Zustand in perspektivischer Ansicht. Fig. 6 zeigt, dass im geschlossenen Zustand der Einrichtung 10 die Abdeckung 46 auf der Aufnahmeschale 40 aufliegt und über die Zugangsöffnungen 36, 60 und die weiteren, nicht bezeichneten bzw. nicht dargestellten Zugangsöffnungen für das Behältnis 16 und das Bedienteil 24 der Stechhilfe 14 ein Blutzuckermessen möglich ist. Ferner ist beispielhaft für die Zugangsöffnung 36 dargestellt, dass die Zugangsöffnung 36 über die beiden Einschnitte der Aufnahmeschale 40 und der Abdeckung 46 gebildet wird. Gleiches gilt für die Zugangsöffnung 60 und die nicht weiter beschriebenen und bezeichneten Zugangsöffnungen für das Behältnis 16 und die Stechhilfe 14.

Wünscht ein Benutzer, seinen Blutzucker zu messen, so kann er die Einrichtung 10 in eine Hand nehmen und mit der anderen Hand den Deckel 42 des Behältnisses 16 öffnen, um einen Teststreifen zu entnehmen. Den Teststreifen kann er anschließend über die Zugangsöffnung 36 in die Zuführöffnung 22 des Blutzuckermessgerätes 12 einsetzen. Anschließend kann er den Deckel 42 des Behältnisses 16 wieder schließen. Nach dem Einsetzen des Teststreifens in die Zuführöffnung 22 wird das Blutzuckermessgerät 12 eingeschaltet oder der Benutzer hat zuvor das Blutzuckermessgerät über einen separaten Knopf, der ebenfalls über eine nicht dargestellte Zugangsöffnung zugänglich ist, eingeschaltet. Nach dem Einschalten des Blutzuckermessgerätes 12 und dem Einsetzen des Teststreifens kann der Benutzer über die Zugangsöffnung durch die Einschnitte 50 über das Bedienteil 24 die Stechhilfe 14 spannen, um anschließend durch eine erneute Betätigung des Bedienteils 24 ein Auslösen der Stechhilfe 14 zu bewirken. Hierbei tritt die Nadel der Lanzette kurzfristig aus der Zugangsöffnung 60 hervor. Wenn ein Benutzer die Einrichtung 10 so hält, dass er beispielsweise mit dem Daumen der linken Hand das Bedienteil 24 sowohl spannen als auch auslösen kann, kann er mit der rechten Hand den Teststreifen entnehmen und in die Zuführöffnung 22 einsetzen sowie einen Finger der rechten Hand vor die Zugangsöffnung 60 halten, um über die Lanzette der Stechhilfe 14 Blut zu gewinnen. Nach dem Stechen kann der Benutzer das aus dem Finger austretende Blut auf einen entsprechenden Testbereich des Teststreifens auftragen, wobei anschließend automatisch die Blutzuckermessung gestartet wird. Nach einer bestimmten Zeit wird das Testergebnis über das Display 20 des Blutzuckermessgerätes 12 dargestellt, wobei über das Fenster 30 der Blick auf das Display 20 möglich ist. Bei dem Ausführungsbeispiel von Fig. 6 wird als Glukosewert beispielsweise 97 mg/dl angezeigt. Nach einem erfolgreichen Messvorgang kann der Benutzer beispielsweise wieder mit der rechten Hand den benutzten Teststreifen aus der Zuführöffnung 22 des Blutzuckermessgerätes 12 entnehmen und entsorgen. Das Blutzuckermessgerät 12 wird anschließend automatisch abgeschaltet oder kann über einen entsprechenden Knopf bzw. eine entsprechende Taste ausgeschaltet werden.

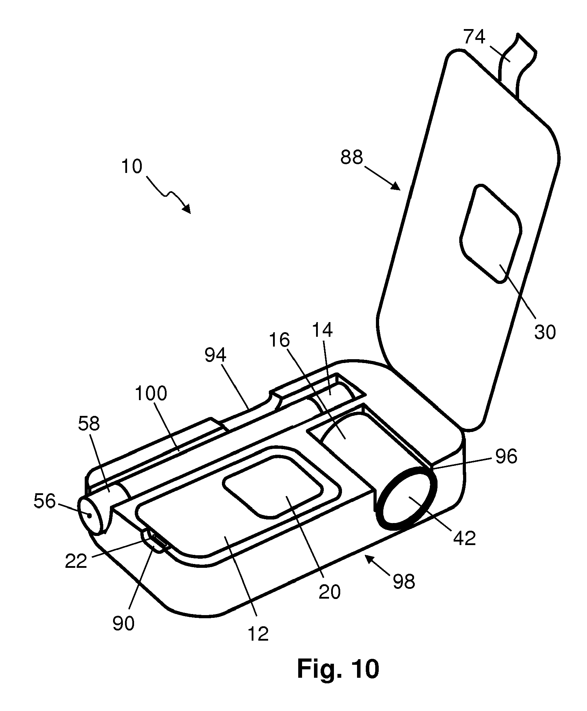

Soll durch die Einrichtung 10 zusätzlich ein Wechsel der Lanzetten erfolgen, ohne die Einrichtung 10 öffnen zu müssen, so kann die Aufnahmeschale 40 entsprechend der in Fig. 10 gezeigten Ausführung ausgebildet sein und größere Einschnitte 54 aufweisen, welche die Aufnahme 58 von außen zugänglich machen, selbst dann, wenn die Einrichtung 10 geschlossen ist.

Fig. 7 zeigt eine schematische Darstellung einer Aufnahmeschale 62 gemäß einer weiteren Ausführung in perspektivischer Ansicht. Die Aufnahmeschale 62 ist als Kunststoffhohlkörper ausgebildet und weist Aufnahmevertiefungen auf, in welchen das Blutzuckermessgerät 12, das Behältnis 16 und die Stechhilfe 14 aufgenommen sind. Von den Aufnahmevertiefungen erstrecken sich Ausbuchtungen 64, 66 und 68, so dass die Zuführöffnung 22 des Blutzuckermessgerätes 12, die Öffnung 56 für die Lanzette und die Öffnung für das Bedienteil 24 der Stechhilfe 14 im geschlossenen Zustand der Einrichtung 10 zugänglich sind. Die Aufnahmevertiefung für das Behältnis 16 weist keine gesonderte Ausbuchtung auf, sondern erstreckt sich bis zum Rand der Aufnahmeschale 62. Damit das Behältnis 16 nicht herausrutschen kann, ist die Aufnahmevertiefung für das Behältnis 16 so ausgebildet, dass das Behältnis 16 unter Klemmwirkung eingesetzt ist. Alternativ dazu können die Aufnahmevertiefung oder das Behältnis 16 eine reibungserhöhende Oberfläche oder ein Gummieinlegeteil oder eine Gummibeschichtung aufweisen. Eine Abdeckung für die Aufnahmeschalte 62 kann entsprechend der Ausführung der Fig. 5 und 6 ausgebildet sein. Alternativ dazu kann die Abdeckung für die Aufnahmeschale 62 entsprechend der Aufnahmeschale 62 als Kunststoffhohlkörper mit Aufnahmevertiefungen ausgebildet sein. Eine derartige Abdeckung weist ebenso Ausbuchtungen auf. Zusätzlich weist die Abdeckung ein Fenster 30 auf, um im geschlossenen Zustand der Einrichtung 10 den Blick auf das Display 20 des Blutzuckermessgerätes 12 freizugeben. Die Abdeckung kann mit der Aufnahmeschale 62 über ein Filmscharnier verschwenkbar verbunden sein und beispielsweise über ein Verschlusselement 74 verschließbar sein.

Alternativ dazu kann sowohl die Abdeckung für die Aufnahmeschale 62 als auch die Aufnahmeschale 62 Rastmittel aufweisen, die ein vollständiges Entnehmen der Abdeckung ermöglichen.

Fig. 8 zeigt eine schematische Darstellung einer weiteren Einrichtung 10 im geöffneten Zustand in perspektivischer Ansicht. Anders als die vorstehend gezeigte Ausführungsform sind die Aufnahmevertiefungen so ausgebildet, dass die Stechhilfe 14, das Blutzuckermessgerät 12 und ein Behältnis 76 vollständig in den Aufnahmevertiefungen aufgenommen sind. Dazu ist bei der Einrichtung 10 eine verschwenkbar mit der Aufnahmeschale 70 verbundene Abdeckung 72 vorgesehen. Die Abdeckung 72 ist flach ausgebildet und weist ein Fenster 30 sowie ein Verschlusselement 74 auf. Die Aufnahmeschale 70 weist Ausbuchtungen 78, 80, 82 und 84 auf. Die Ausbuchtung 78 ermöglicht im geschlossenen Zustand der Einrichtung 10 einen Zugang zu der Zuführöffnung 22 des Blutzuckermessgerätes 12. Die Ausbuchtung 80 ermöglicht ein Hervortreten der Lanzette der Steckhilfe 14 im geschlossenen Zustand der Einrichtung 10 und die Ausbuchtung 82 ermöglicht ein Bedienen des Bedienteils 24 der Stechhilfe 14 im geschlossenen Zustand der Einrichtung 10. Über die Ausbuchtung 84 können aus dem rechteckig ausgebildeten Behältnis 76 im geschlossenen Zustand der Einrichtung 10 Teststreifen entnommen werden.

In einer alternativen Ausführungsform weist die Einrichtung 10 von Fig. 8 keine breite Ausbuchtung 84 auf, sondern lediglich eine dünne schlitzartige Ausbuchtung, über welche jeweils nur ein Teststreifen aus dem Behältnis 76 entnehmbar ist. Hierzu weist die Einrichtung 10 eine Zugangsöffnung für einen Schieber des Behältnisses 76 auf. Der Zugang für den Schieber kann entweder in der Abdeckung 72 oder in der Aufnahmeschale 70 vorgesehen sein.

Die flache Abdeckung 72 kann aus Kunststoff oder Leder gefertigt sein. Zudem kann die Aufnahmeschale 70 auch einen Lederüberzug aufweisen.

In weiteren Ausführungsformen können Aufnahmeschalen aus Kunststoff in einer Ledertasche aufgenommen sein, welche zudem eine flache Abdeckung aufweist.

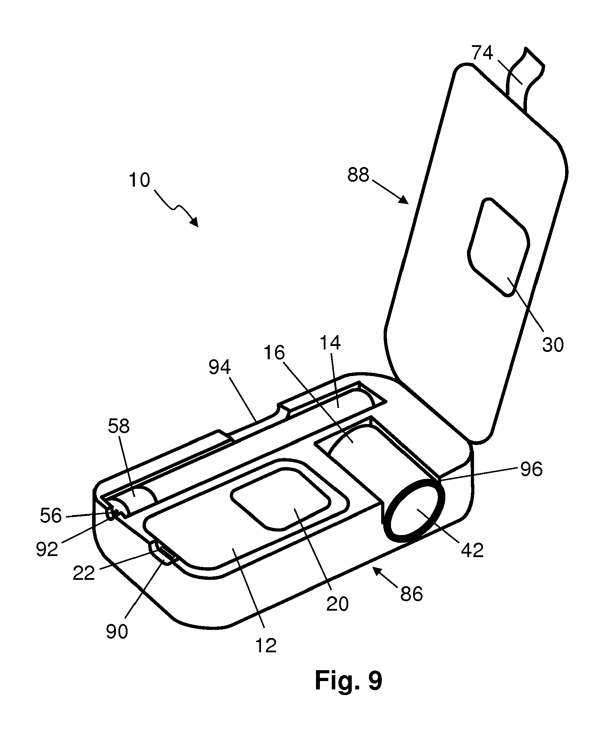

Fig. 9 zeigt eine schematische Darstellung einer weiteren Einrichtung 10 im geöffneten Zustand in perspektivischer Ansicht.

Die Einrichtung 10 von Fig. 9 unterscheidet sich von der Ausführung von Fig. 8 dadurch, dass anstelle eines rechteckigen Behältnisses 76 ein rundes Behältnis 16 aufgenommen ist. Die Aufnahmeschale 86 von Fig. 9 unterscheidet sich daher in der Höhe der Aufnahmeschale 70 von Fig. 8. Auch die Aufnahmeschale 86 von Fig. 9 besteht aus einem Kunststoffhohlkörper mit Aufnahmevertiefungen für das Blutzuckermessgerät 12, die Stechhilfe 14 und das Behältnis 16. Von den Aufnahmevertiefungen erstrecken sich Ausbuchtungen 90, 92, 94 und 96 zu den Außenseiten der Aufnahmeschale 86. Die Ausbuchtung 96 der Aufnahmevertiefung für das Behältnis 16 entspricht im Wesentlichen der Aufnahmevertiefung für das Behältnis 16. Die Ausbuchtungen 90, 92 und 94 geben den Zugang zu dem Blutzuckermessgerät 12 und den funktionsrelevanten Bereichen der Stechhilfe 14 im geschlossenen Zustand der Einrichtung 10 frei. Im geschlossenen Zustand der Einrichtung 10 liegt die Abdeckung 88 auf der Aufnahmeschale 86 auf. Die Abdeckung 88 weist ein Fenster 30 auf, über welches das Display 20 des Blutzuckermessgerätes 12 im geschlossenen Zustand der Einrichtung 10 ersichtlich ist. Über das Verschlusselement 74 kann die Einrichtung 10 verschlossen werden.

Fig. 10 zeigt eine schematische Darstellung einer Ausführungsvariante einer Einrichtung 10 im geöffneten Zustand der Ausführung von Fig. 9 in perspektivischer Ansicht. In der Ausführungsvariante der Einrichtung 10 von Fig. 10 ist die Aufnahme 58 der Stechhilfe 14 aus der Aufnahme 100 auch im geschlossenen Zustand der Einrichtung 10 zugänglich. Die Aufnahme 58 kann im geschlossenen Zustand der Einrichtung 10 von der Stechhilfe 14 entnommen werden. Dadurch können die Lanzetten für die Stechhilfe 14 ausgetauscht werden.

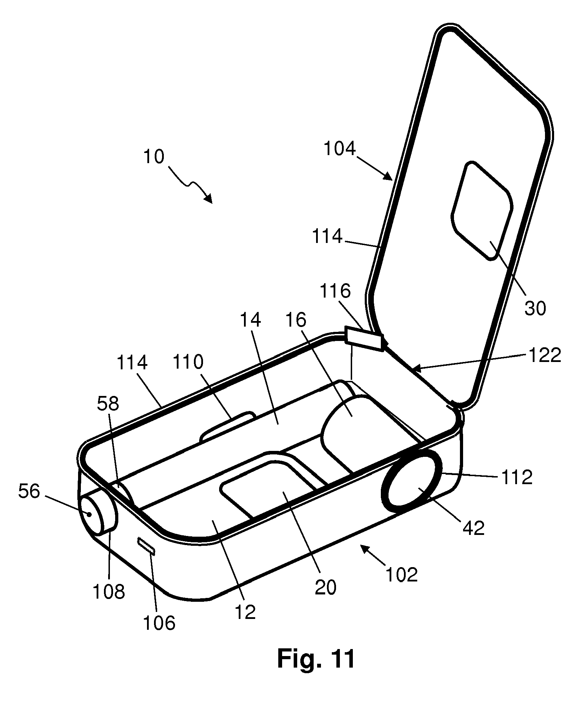

Fig. 11 zeigt eine schematische Darstellung einer weiteren Ausführungsform einer Einrichtung 10 im geöffneten Zustand in perspektivischer Ansicht. Die Einrichtung 10 besteht aus einem Unterteil 102 und einem Oberteil 104. Das Oberteil 104 und das Unterteil 102 bestehen aus einem flexiblen Material, wobei das Unterteil 102 zur Verstärkung eine Bodenplatte aus Kunststoff aufweist. An der Bodenplatte des Unterteils 102 sind das Blutzuckermessgerät 12, die Stechhilfe 14 und das Behältnis 16 über nicht dargestellte Halteteile 26 befestigt. Das Unterteil 102 weist Öffnungen 106, 108, 110 und 112 auf. Über die Öffnung 106 ist die Zuführöffnung 22 des Blutzuckermessgerätes 12 von außen im geschlossenen Zustand der Einrichtung 10 zugänglich. Über die Öffnung 108 ist die Aufnahme 58 der Stechhilfe 14 auch im geschlossenen Zustand der Einrichtung 10 zugänglich. Die Aufnahme 58 kann im geschlossenen Zustand der Einrichtung 10 von der Stechhilfe 14 abgezogen werden, um eine neue Lanzette einzusetzen oder eine alte Lanzette zu entfernen. Ferner weist das Unterteil 102 eine Öffnung 110 auf, über die das in Fig. 11 nicht dargestellte Bedienteil 24 der Stechhilfe 14 zum Spannen und Auslösen zugänglich ist. Ferner weist das Unterteil 102 eine Öffnung 112 auf, aus der der Deckel 42 des Behältnisses 16 auch im geschlossenen Zustand der Einrichtung 10 herausragt und damit eine Entnahme von Teststreifen aus dem Behältnis 16 ermöglicht. Das Oberteil 104 weist ein Fenster 30 auf, welches im geschlossenen Zustand der Einrichtung 10 den Blick auf das Display 20 des Blutzuckermessgerätes 12 freigibt. Das Unterteil 102 und das Oberteil 104 sind über die Kante 122 zueinander verschwenkbar. Über den Reißverschluss 114 und den Zipper 116 können das Unterteil 102 und das Oberteil 104 miteinander verbunden werden, um die Einrichtung 10 zu schließen.

Anders als in den Fig. 1 bis 11 dargestellt, können in weiteren Ausführungsformen die Aufnahmeteile, Aufnahmeschalen und Abdeckungen bzw. Unterteil und Oberteil rechteckig ausgeführt sein.

### Bezugszeichenliste

- 10: Einrichtung
- 12: Blutzuckermessgerät
- 14: Stechhilfe
- 16: Behältnis
- 18: Bereich
- 20: Display
- 22: Zuführöffnung
- 24: Bedienteil
- 26: Halteteil
- 28: Gummiband
- 30: Fenster
- 32: Aufnahmeschale
- 34: Abdeckung
- 36: Zugangsöffnung
- 38: Aufnahmeboden
- 40: Aufnahmeschale
- 42: Deckel
- 44: Filmscharnier
- 46: Abdeckung
- 48: Einschnitt
- 50: Einschnitt
- 52: Einschnitt
- 54: Einschnitt
- 56: Öffnung
- 58: Aufnahme
- 60: Zugangsöffnung
- 62: Aufnahmeschale
- 64: Ausbuchtung
- 66: Ausbuchtung
- 68: Ausbuchtung
- 70: Aufnahmeschale
- 72: Abdeckung
- 74: Verschlusselement
- 76: Behältnis
- 78: Ausbuchtung
- 80: Ausbuchtung
- 82: Ausbuchtung
- 84: Ausbuchtung
- 86: Aufnahmeschale
- 88: Abdeckung
- 90: Ausbuchtung
- 92: Ausbuchtung
- 94: Ausbuchtung
- 96: Ausbuchtung
- 98: Aufnahmeschale
- 100: Aufnahme
- 102: Unterteil
- 104: Oberteil
- 106: Öffnung
- 108: Öffnung
- 110: Öffnung
- 112: Öffnung
- 114: Reißverschluss
- 116: Zipper
- 118: Kante
- 120: Kante
- 122: Kante

## Patentansprüche

1. Einrichtung zur Aufbewahrung von Utensilien zur Blutzuckermessung für Diabetiker, wobei die Utensilien mindestens ein Blutzuckermessgerät (12) mit einer Aufnahme für Teststreifen, eine Stechhilfe (14) mit auswechselbaren Lanzetten und ein Behältnis (16; 76) für Teststreifen umfassen, **dadurch gekennzeichnet, dass** die Einrichtung (10) Bereiche (18) zur Aufnahme mindestens der vorgenannten Utensilien aufweist, wobei die Utensilien in den Bereichen (18) mit der Einrichtung (10) lösbar befestigt und die Utensilien über mindestens eine Seite der Einrichtung (10) funktional zugänglich sind, wobei die Einrichtung (10) an der mindestens einen Seite Zugangsbereiche aufweist, die im Wesentlichen kongruent zu Zuführund Entnahmebereichen der Utensilien sind und eine Bedienung der Utensilien ohne eine Entnahme der Utensilien ermöglichen.

2. Einrichtung nach Anspruch 1, wobei die Einrichtung (10) ein Aufnahmeteil und eine Abdeckung (34; 46; 72; 88) aufweist und die Bereiche (18) zur Aufnahme der Utensilien in dem Aufnahmeteil angeordnet sind, wobei das Aufnahmeteil und die Abdeckung (34; 46; 72; 88) mindestens abschnittsweise lösbar miteinander verbunden und die Utensilien über Aufnahmen in dem Aufnahmeteil befestigt sind, wobei die Zugangsbereiche in Wänden der Aufnahmen des Aufnahmeteils und/oder der Abdeckung (34; 46; 72; 88) angeordnet sind.

3. Einrichtung nach Anspruch 1 oder 2, wobei das Aufnahmeteil aus Kunststoff besteht oder als Kunststoffvolloder hohlkörper ausgebildet ist und schalenförmige Aufnahmen für die Utensilien aufweist, die darin klemmend haltbar sind.

4. Einrichtung nach Anspruch 2 oder 3, wobei das Aufnahmeteil eine ebene Grundfläche und eine der höchsten Utensilie entsprechende Höhe oder eine inhomogene Oberfläche mit einer den Höhen der Utensilien angepassten Abstufung aufweist.

5. Einrichtung nach Anspruch 2 oder 3, wobei das Aufnahmeteil und/oder die Abdeckung (34; 46; 70; 88) aus einem flexiblen Material bestehen oder die Wände der schalenförmigen Aufnahmen federelastisch ausgeführt sind.

6. Einrichtung nach Anspruch 5, wobei das Aufnahmeteil mindestens eine Verstärkung aufweist.

7. Einrichtung nach einem der Ansprüche 2 bis 6, wobei das Aufnahmeteil und/oder die Abdeckung (34; 46; 70; 88) Befestigungsmittel und/oder Aufnahmevertiefungen für die Utensilien aufweisen.

8. Einrichtung nach einem der Ansprüche 2 bis 7, wobei das Aufnahmeteil und/oder die Abdeckung (34; 46; 70; 88) eine Öffnung aufweisen, über welche im geschlossenen Zustand der Einrichtung (10) mindestens eine Anzeige des Blutzuckermessgeräts sichtbar ist.

9. Einrichtung nach einem der Ansprüche 1 bis 8, wobei die Einrichtung (10) Befestigungsmittel aufweist, über welche die Einrichtung (10) an Textilien oder Gegenständen lösbar befestigbar ist.

10. Einrichtung nach einem der Ansprüche 7 bis 9, wobei die Größe und der Umfang der Aufnahmevertiefungen jeweils an das Maß und den Umfang einer der Utensilien angepasst sind.

11. Einrichtung nach einem der Ansprüche 2 bis 10, wobei das Aufnahmeteil und die Abdeckung (34; 46; 70; 88) über einen Magnetverschluss, eine Rastverbindung, einen Klettverschluss oder einen Reißverschluss (114) mindestens abschnittsweise miteinander verbindbar sind.

12. Einrichtung nach einem der Ansprüche 2 bis 11, wobei das Aufnahmeteil und die Abdeckung (34; 46; 70; 88) verschwenkbar miteinander verbunden sind.

13. Einrichtung nach einem der Ansprüche 1 bis 12, wobei die Zugangsbereiche Öffnungen (106, 108, 110, 112), Ausnehmungen oder Durchbrüche sind.

14. Einrichtung nach einem der Ansprüche 1 bis 13, wobei die Einrichtung zusätzliche Bereiche zur Aufnahme weiterer Utensilien aufweist.

15. Einrichtung nach Anspruch 14, wobei die weitere Utensilie ein Insulinspritzgerät (Pen) ist, das ohne eine Schutzkappe in eine röhrenförmige Aufnahme der Einrichtung einsteckbar und aus dieser wahlweise wieder herausziehbar ist, und das mit einer Schutzkappe mindestens mit dieser in einer Aufnahme einklemmbar eingelegt ist und aus der Schutzkappe wahlweise herausziehbar ist.
